# EUROPEAN PATENT SPECIFICATION

(11) **EP 4 103 543 B1**
(45) Date of publication and mention of the grant of the patent: **06.12.2023**
(21) Application number: 21705448.5
(22) Date of filing: 10.02.2021
(51) Int. Cl.: C07C 231/02, C07C 231/16, C07C 51/487, C07C 51/41, C07B 53/00

(54) **PROCESS FOR THE SYNTHESIS OF S-BEFLUBUTAMID USING ASYMMETRIC HYDROGENATION**
VERFAHREN ZUR HERSTELLUNG VON OF-BEFLUTAMID UNTER VERWENDUNG EINER ASYMMETRISCHEN HYDRIERUNG
PROCÉDÉ DE SYNTHÈSE DE-BÉFLUBUTAMIDE À L'AIDE D'UNE HYDROGÉNATION ASYMÉTRIQUE

(30) Priority: 11.02.2020 US 202062972779 P
(43) Date of publication of application: 21.12.2022
(73) Proprietor: Cheminova A/S, 7673 Ronland (DK)
(72) Inventor: SØNDERGAARD, Kåre, Philadelphia, Pennsylvania 19104 (US)
(74) Representative: Dehns
(86) International application number: PCT/EP2021/053159
(87) International publication number: WO 2021/160649

(56) References cited:
- EP-A1- 0 239 414
- CHENG XU ET AL: "Asymmetric Hydrogenation of [alpha],[beta]-Unsaturated Carboxylic Acids Catalyzed by Ruthenium(II) Complexes of Spirobifluorene Diphosphine (SFDP) Ligands", ADVANCED SYNTHESIS AND CATALYSIS, vol. 348, no. 10-11, 1 July 2006 (2006-07-01), pages 1271-1276, XP55797094, ISSN: 1615-4150, DOI: 10.1002/adsc.200606065

## Description

### FIELD OF THE INVENTION

This invention relates to a method for preparing the S-enantiomer of beflubutamid.

### BACKGROUND OF THE INVENTION

U.S. Patent No. 4,929,273 discloses N-benzyl-2-(4-fluoro-3-trifluoromethylphenoxy)-butanoic amide of Formula **1** as an herbicidal compound. It has a single asymmetric center at the 2-carbon of the amide moiety and thus can be a chiral molecule.

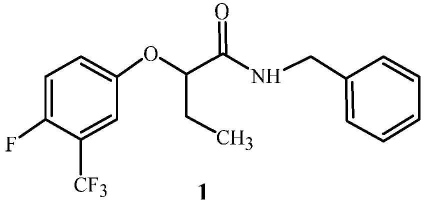

This compound in racemic form has been marketed commercially under the common name beflubutamid as a soil herbicide for pre- and post-emergence control of dicotyledonous weeds in cereals. It inhibits the enzyme phytoene-desaturase that is involved in the biosynthesis of carotenoids. Depletion of carotenoids leads to photooxidation of chlorophyll and bleaching/chlorosis of susceptible weeds.

U.S. Patent No. 4,929,273 also discloses that the (-)-optical isomer is more herbicidally active than the racemic mixture. The more active enantiomer has been identified as having the S-configuration shown as compound ***S*-1** (Environ. Sci. Technol. 2013, 47, 6806-6811 and Environ. Sci. Technol. 2013, 47, 6812-6818).

Further processes towards beflubutamid are known from EP0239414 and Cheng et al in Advanced Synthesis and Catalysis, 2006, 348(10-11), 1271-1276.

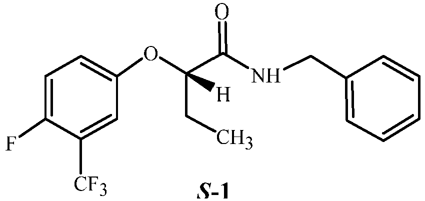

While the methods disclosed in the preceding references can provide desired compound ***S*-1,** continuous improvements are sought, particularly in the development of methods to provide materials on a commercial scale. Therefore, the need continues for new methods that are less costly, more efficient, more flexible, or more convenient to operate.

### SUMMARY OF THE INVENTION

### Embodiment A.

This invention provides a method for preparing compound ***S*-1** 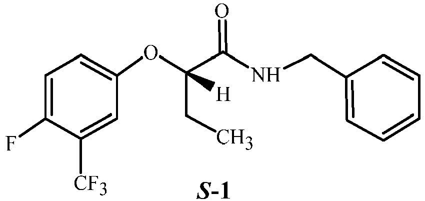 from compound ***S*-5** 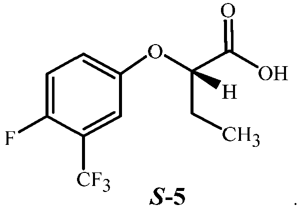 wherein compound ***S*-5** is prepared by treating compound **2** 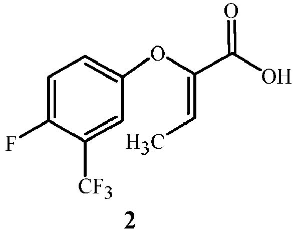 with a tertiary amine and a hydrogen source in the presence of a chiral complex.

### Embodiment B.

This invention also provides a method for preparing compound ***S*-1** 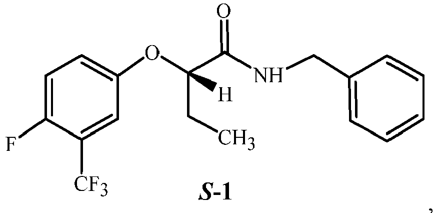 the method comprising:
treating compound **2** 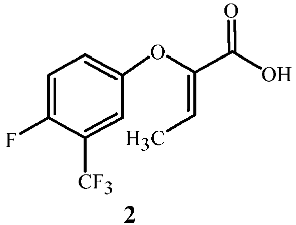 with a tertiary amine and a hydrogen source in the presence of a chiral complex to prepare compound *S*-5 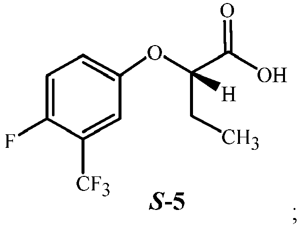 and converting compound ***S*-5** to compound ***S*-1.**

### Embodiment C.

This invention also provides a method for preparing compound ***S*-1** 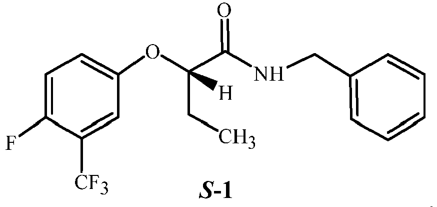 the method comprising:
treating a compound of Formula **6** 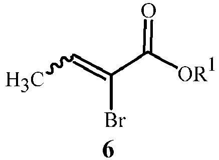 wherein R¹ is C₁-C₆ alkyl;
with compound 7 (i.e. 4-fluoro-3-(trifluoromethyl)phenol) 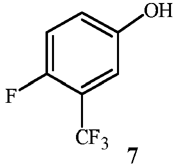 in the presence of a base to provide a compound of Formula **8** 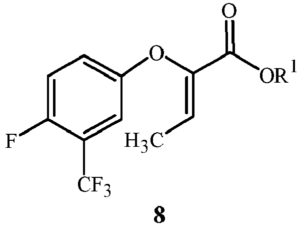 wherein R¹ is C₁-C₆ alkyl; hydrolyzing the compound of Formula **8** to provide compound **2** 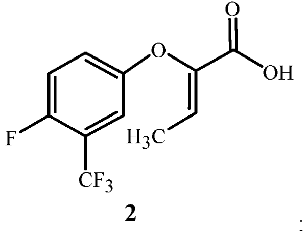 treating compound **2** with a tertiary amine and a hydrogen source in the presence of a chiral complex to prepare compound ***S*-5** 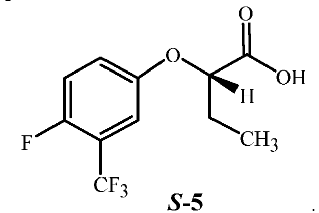 and converting compound ***S*-5** to the compound of Formula ***S*-1.**

### Embodiment D.

This invention also provides a method for preparing compound ***S*-5** 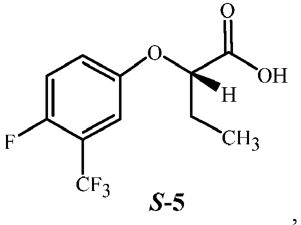 the method comprising:
treating compound **2** 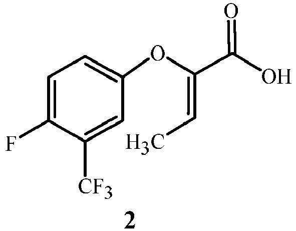 with a tertiary amine and a hydrogen source in the presence of a chiral complex.

### Embodiment E.

This invention also provides a compound of Formula **16** 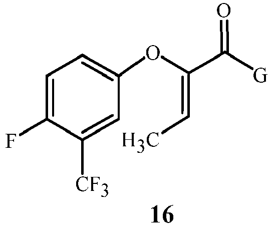
wherein G is OH, C₁-C₆ alkoxy or O⁻ M⁺; and
M⁺ is an alkali metal cation or a tertiary ammonium cation.

### DETAILED DESCRIPTION OF THE INVENTION

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process or method.

The transitional phrase "consisting of" excludes any element, step, or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of" is used to define a composition, process or method that includes materials, steps, features, components, or elements, in addition to those literally disclosed, provided that these additional materials, steps, features, components, or elements do not materially affect the basic and novel characteristic(s) of the claimed invention. The term "consisting essentially of" occupies a middle ground between "comprising" and "consisting of'.

Where applicants have defined an invention or a portion thereof with an open-ended term such as "comprising," it should be readily understood that (unless otherwise stated) the description should be interpreted to also describe such an invention using the terms "consisting essentially of" or "consisting of."

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

As used herein, the term "suitable" indicates that the entity or condition so described is appropriate for use in the situation or circumstance indicated. As used herein, the terms "treatment" or treating" denotes using a chemical or chemical process to alter the existing condition of other materials, chemicals or compounds. The term "converting" refers to causing an entity such as a chemical compound to change in structure, form, character or function. For example, a compound of a first formula or structure is converted to a compound of a second formula or structure by a chemical process involving one or more treatments as defined above.

In the above recitations, the term "alkyl", used either alone or in compound words such as "alkylthio" or "haloalkyl" includes straight-chain or branched alkyl, such as methyl, ethyl, n-propyl, i-propyl, or the different butyl, pentyl or hexyl isomers.

"Alkoxy" includes, for example, methoxy, ethoxy, n-propyloxy, isopropyloxy and the different butoxy, pentoxy and hexyloxy isomers.

As used herein, "alkali metal" refers to elements of group 1 of the periodic table, including lithium, sodium, potassium and cesium, preferably sodium or potassium, or cations thereof, such as when used in combination with an anionic counterion to define a chemical compound. "Alkaline earth metal" refers to elements of group 2 of the periodic table, including magnesium, calcium, or cations thereof, such as when used in combination with an anionic counterion to define a chemical compound.

The term "tertiary ammonium cation" refers to a protonated tertiary amine species, for example, a triethylammonium cation, (ethyl)₃NH⁺.

The term "halogen", either alone or in compound words such as "haloalkyl", or when used in descriptions such as "halogenation" includes fluorine, chlorine, bromine or iodine.

The total number of carbon atoms in a substituent group is indicated by the "Cᵢ-Cⱼ" prefix where i and j are numbers from 1 to 6.

The term "optionally" when used herein means that the optional condition may or may not be present. For example, when a reaction is conducted optionally in the presence of a solvent, the solvent may or may not be present.

As used herein, the terms "hydrolysis", "hydrolyzing" and related terms refer to treating a compound with water to rupture one or more bonds to transform the compound to another compound. For example, an ester, an amide, an acid chloride or a carboxylate salt can be treated with water (hydrolyzed) to obtain the corresponding carboxylic acid. The hydrolysis reaction can be conducted under neutral, acidic or basic conditions depending on the transformation desired.

This invention includes compounds, or methods of preparing compounds, that are enantiomerically enriched compared to a racemic mixture; for example in an enantiomer of compound ***S*-1** or any intermediate in a process described herein for preparing compound ***S*-1.** The compound ***S*-1** or any intermediate in a process described herein for preparing compound ***S*-1** may also be in the form of an essentially pure enantiomer. The basic and novel characteristics of this invention include preparation of compounds ***S*-1** and ***S*-5** by treating compound **2** with a tertiary amine and a hydrogen source in the presence of a chiral complex as described herein. The basic and novel characteristics of this invention also include any compound of Formula **16** as described herein, including any compositions containing compound of Formula **16.**

When enantiomerically enriched, one enantiomer is present in greater amounts than the other, and the extent of enrichment can be defined by an expression of enantiomeric excess ("ee"), which is defined as (*F*_{m*a*j} - *F*ₘᵢₙ)·100%, where *F*ₘₐⱼ is the mole fraction of the dominant enantiomer in the mixture and *F*ₘᵢₙ is the mole fraction of the lesser enantiomer in the mixture (e.g., an ee of 20% corresponds to a 60:40 ratio of enantiomers).

As used herein, compounds having at least an 80% enantiomeric excess; preferably at least a 90 % enantiomeric excess; more preferably at least a 94% enantiomeric excess, at least a 96% enantiomeric excess, or at least a 98% enantiomeric excess of a specific isomer are designated as *R*- or *S*-, depending on the predominant configuration at the asymmetric center. Of note are essentially enantiomerically pure embodiments (>99% ee) of the more predominant enantiomer. As used herein, compounds having less than 80% enantiomeric excess are designated as scalemic.

Molecular depictions drawn herein generally follow standard conventions for depicting stereochemistry. To indicate stereoconfiguration, bonds rising from the plane of the drawing and towards the viewer are denoted by solid wedges where the broad end of the wedge is attached to the atom rising from the plane of the drawing towards the viewer as shown below, where group B is rising from above the plane of the drawing. Except where specifically indicated, hydrogen atoms attached to an asymmetric center are generally not shown.

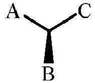

Bonds going below the plane of the drawing and away from the viewer are denoted by dashed wedges where the broad end of the wedge is attached to the atom further away from the viewer, i.e. group B' is below the plane of the drawing.

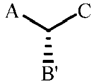

Constant width lines indicate bonds with a direction opposite or neutral relative to bonds shown with solid or dashed wedges; constant width lines also depict bonds in molecules or parts of molecules in which no stereoconfiguration is intended to be specified. Notably as used herein, a constant width line attached to an asymmetric center also represents a condition where the amounts of *R*- and *S*-configuration at that center are equal; e.g., a compound with a single asymmetric center is racemic. 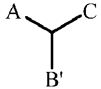 Racemic mixture or "**rac"**

Wavy lines indicate bonds in molecules or parts of molecules in which no particular stereoconfiguration is intended to be specified. As used herein, a wavy line attached to an alkenyl carbon represents a condition wherein the amounts of *E*- and *Z*-configuration at that carbon are not defined; for example, the configuration at that carbon may be a mixture of *E*- and *Z*-configuration. 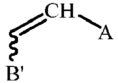 mixture of *E*- and *Z*-configurtion

Embodiments of the invention include the following.

Embodiment A1. The method of Embodiment A wherein the chiral complex comprises ruthenium complexed with a chiral bisphosphine.

Embodiment A2. The method of Embodiment A1 wherein the chiral complex comprises a dichloro ruthenium complex with a chiral bisphosphine.

Embodiment A3. The method of Embodiment A3 wherein the chiral complex comprises a dichloro ruthenium complex with an asymmetric atropisomeric bisphosphine.

Embodiment A4. The method of any of Embodiments A, A1, A2 or A3 wherein the bisphosphine ligand is selected from the group consisting of (*S*)-synphos, (*S*)-P-phos, (*S*)-Cl-MeO-BIPHEP, (-)-TMBTP and (*S*)-BINAP.

Embodiment A5. The method of Embodiment A4 wherein the chiral complex is chloro[(*S*)-(-)-2,2'-bis(diphenylphosphino)-1,1'-binaphthyl](*p*-cymene)ruthenium(II) chloride, i.e. (*S*)-BINAP.

Embodiment A6. The method of any of Embodiments A through A5 wherein the tertiary amine comprises triethylamine, diisopropylethylamine, *N*,*N*,*N'*,*N'*-tetramethylethylenediamine, *N-*methylpiperidine, *N*-phenylpiperidine or *N*-methylmorpholine.

Embodiment A7. The method of Embodiment A6 wherein the tertiary amine comprises triethylamine.

Embodiment A8. The method of any of Embodiments A through A7 wherein compound ***S*-5** is prepared by the method comprising treating compound **2** with triethylamine to provide the triethylamine salt **3** 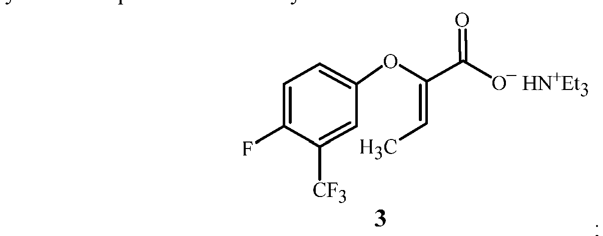 treating the triethylamine salt **3** with a hydrogen source in the presence of a chiral complex to provide triethylamine salt ***S*-4** 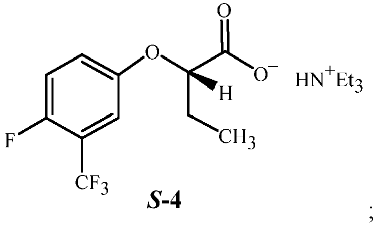 and treating the triethylamine salt of Formula ***S*-4** with acid.

Embodiment A9. The method of any of Embodiments A through A8 wherein compound **2** is prepared by treating a compound of Formula **6** 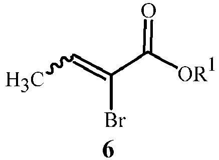 wherein R¹ is C₁-C₆ alkyl;
with compound **7** 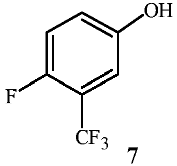 in the presence of a base to provide a compound of Formula **8** 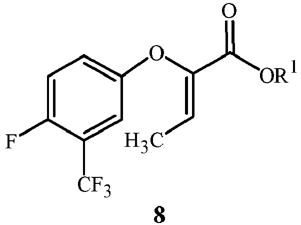 wherein R¹ is C₁-C₆ alkyl; and
hydrolyzing the compound of Formula **8.**

Embodiment A10. The method of Embodiment A9 wherein R¹ is CH₃.

Embodiment A11. The method of Embodiment A10 wherein the base is a hydride, alkoxide, hydroxide, carbonate, bicarbonate of an alkali metal or an alkaline earth metal, or an amide base, or a tertiary amine.

Embodiment A12. The method of Embodiment A11 wherein the base is sodium hydroxide, potassium hydroxide, sodium bicarbonate, potassium bicarbonate, sodium carbonate or potassium carbonate.

Embodiment A13. The method of Embodiment A12 wherein the base is in an aqueous solution.

Embodiment A14. The method of Embodiment A12 or A13 wherein the base is potassium carbonate.

Embodiment A15. The method of any of Embodiments A through A14 wherein preparing compound ***S*-1** from compound ***S*-5** comprises
treating compound ***S*-5** with a chlorinating agent to prepare compound ***S*-9** 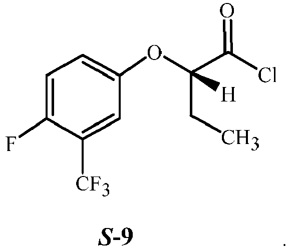 and treating compound ***S*-9** optionally in the presence of an additional base with compound **10** (i.e. benzylamine)

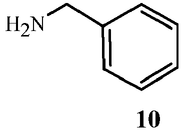

Embodiment A16. The method of Embodiment A15 wherein the chlorinating agent is POCl₃, SOCl₂, (COCl)₂ or COCl₂.

Embodiment A17. The method of Embodiment A16 wherein the chlorinating agent is thionyl chloride, i.e. SOCl₂.

Embodiment A18. The method of any of Embodiments A14 through A17 wherein the additional base comprises a hydride, alkoxide, hydroxide, carbonate, bicarbonate of an alkali metal or an alkaline earth metal; or an amide base; or a tertiary amine.

Embodiment A19. The method of Embodiment A18 wherein the additional base comprises triethylamine, sodium hydroxide, potassium hydroxide, sodium bicarbonate, potassium bicarbonate, sodium carbonate or potassium carbonate.

Embodiment A20. The method of Embodiment A19 wherein the additional base comprises triethylamine.

Embodiment B1. The method of Embodiment B wherein the chiral complex comprises ruthenium complexed with a chiral bisphosphine.

Embodiment B2. The method of Embodiment B1 wherein the chiral complex comprises a dichloro ruthenium complex with a chiral bisphosphine.

Embodiment B3. The method of Embodiment B2 wherein the chiral complex comprises a dichloro ruthenium complex with an asymmetric atropisomeric bisphosphine.

Embodiment B4. The method of any of Embodiments B through B3 wherein the bisphosphine ligand is selected from the group consisting of (*S*)-synphos, (*S*)-P-phos, (*S*)-Cl-MeO-BIPHEP, (-)-TMBTP and (*S*)-BINAP.

Embodiment B5. The method of any of Embodiments B through B4 wherein the chiral complex is chloro[(*S*)-(-)-2,2'-bis(diphenylphosphino)-1,1'-binaphthyl](*p-*cymene)ruthenium(II) chloride, i.e. (*S*)-BINAP.

Embodiment B6. The method of any of Embodiments B through B5 wherein the tertiary amine comprises triethylamine, diisopropylethylamine, *N*,*N*,*N'*,*N'*-tetramethylethylenediamine, *N-*methylpiperidine, *N*-phenylpiperidine or *N*-methylmorpholine.

Embodiment B7. The method of Embodiment B6 wherein the tertiary amine comprises triethylamine.

Embodiment B8. The method of any of Embodiments B through B7 comprising treating compound **2** with triethylamine to provide the triethylamine salt of Formula **3** 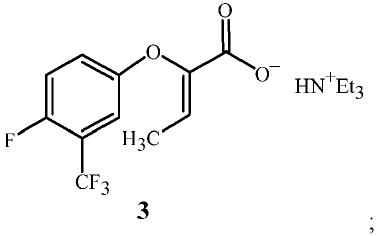 treating the triethylamine salt of Formula **3** with a hydrogen source in the presence of a chiral complex to provide triethylamine salt ***S*-4** 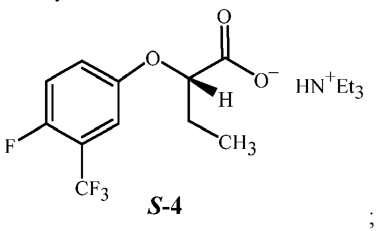 and treating triethylamine salt ***S*-4** with acid to provide compound ***S*-5.**

Embodiment B9. The method of any of Embodiments B through B8 wherein the compound of Formula **2** is prepared by treating a compound of Formula **6** 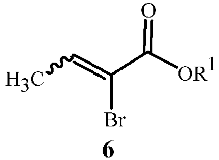 wherein R¹ is C₁-C₆ alkyl;
with compound **7** 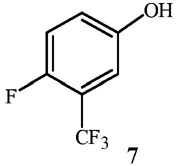 in the presence of a base to provide a compound of Formula **8** 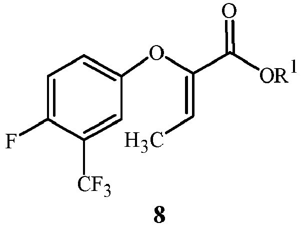 wherein R¹ is C₁-C₆ alkyl; and
hydrolyzing the compound of Formula **8.**

Embodiment B10. The method of Embodiment B9 wherein R¹ is CH₃.

Embodiment B11. The method of Embodiment B10 wherein the base is a hydride, alkoxide, hydroxide, carbonate, bicarbonate of an alkali metal or an alkaline earth metal; or an amide base; or a tertiary amine.

Embodiment B12. The method of Embodiment B11 wherein the base is sodium hydroxide, potassium hydroxide, sodium bicarbonate, potassium bicarbonate, sodium carbonate or potassium carbonate.

Embodiment B13. The method of Embodiment B12 wherein the base is in an aqueous solution.

Embodiment B14. The method of Embodiment B12 or B13 wherein the base is potassium carbonate.

Embodiment B15. The method of any of Embodiments B through B14 wherein converting compound ***S*-5** to compound ***S*-1** comprises
treating compound ***S*-5** with a chlorinating agent to prepare compound ***S*-9** 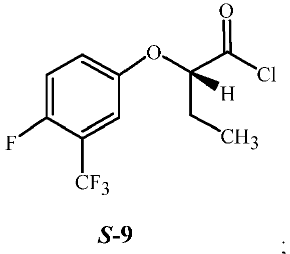 and treating compound ***S*-9** optionally in the presence of an additional base with compound **10**

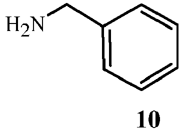

Embodiment B16. The method of Embodiment B15 wherein the chlorinating agent is POCl₃, SOCl₂, (COCl)₂ or COCl₂.

Embodiment B17. The method of Embodiment B16 wherein the chlorinating agent is thionyl chloride, i.e. SOCl₂.

Embodiment B18. The method of any of Embodiments B15 through B17 wherein the additional base comprises a hydride, alkoxide, hydroxide, carbonate, bicarbonate of an alkali metal or an alkaline earth metal; or an amide base; or a tertiary amine.

Embodiment B19. The method of Embodiment B18 wherein the additional base comprises triethylamine, sodium hydroxide, potassium hydroxide, sodium bicarbonate, potassium bicarbonate, sodium carbonate or potassium carbonate.

Embodiment B20. The method of Embodiment B19 wherein the additional base comprises triethylamine.

Embodiment C1. The method of Embodiment C wherein R¹ is CH₃.

Embodiment C2. The method of Embodiment C or C1 wherein the base is a hydride, alkoxide, hydroxide, carbonate, bicarbonate of an alkali metal or an alkaline earth metal, or an amide base, or a tertiary amine.

Embodiment C3. The method of Embodiment C2 wherein the base is sodium hydroxide, potassium hydroxide, sodium bicarbonate, potassium bicarbonate, sodium carbonate or potassium carbonate.

Embodiment C4. The method of Embodiment C3 wherein the base is in an aqueous solution.

Embodiment C5. The method of Embodiment C3 or C4 wherein the base is potassium carbonate.

Embodiment C6. The method of any of Embodiments C through C5 wherein the chiral complex comprises ruthenium complexed with a chiral bisphosphine.

Embodiment C7. The method of Embodiments C6 wherein the chiral complex comprises a dichloro ruthenium complex with a chiral bisphosphine.

Embodiment C8. The method of Embodiment C7 wherein the chiral complex comprises a dichloro ruthenium complex with an asymmetric atropisomeric bisphosphine.

Embodiment C9. The method of any of Embodiments C6 through C8 wherein the bisphosphine ligand is selected from the group consisting of (*S*)-synphos, (*S*)-P-phos, (*S*)-Cl-MeO-BIPHEP, (-)-TMBTP and (*S*)-BINAP.

Embodiment C10. The method of Embodiment C9 wherein the bisphosphine ligand is 2'-bis(diphenylphosphino)-1,1'-binaphthyl](*p*-cymene)ruthenium(II) chloride, i.e. (*S*)-BINAP.

Embodiment C11. The method of any of Embodiments C through C10 wherein the tertiary amine comprises triethylamine, diisopropylethylamine, *N*,*N*,*N'*,*N'*-tetramethylethylenediamine, *N-*methylpiperidine, *N*-phenylpiperidine or *N*-methylmorpholine.

Embodiment C12. The method of Embodiment C11 wherein the tertiary amine comprises triethylamine.

Embodiment C13. The method of any of Embodiments C through C12 comprising treating compound **2** with triethylamine to provide the triethylamine salt of Formula **3** 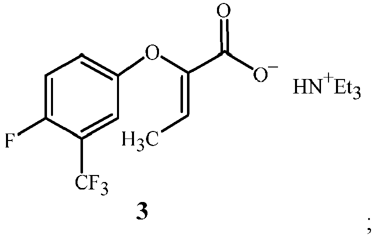 treating the triethylamine salt of Formula **3** with a hydrogen source in the presence of a chiral complex to provide the triethylamine salt of Formula ***S*-4** 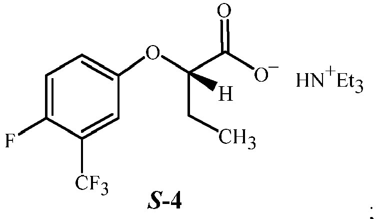 and treating the triethylamine salt of Formula ***S*-4** with acid to provide compound ***S*-5.**

Embodiment C14. The method of any of Embodiments C through C13 wherein converting compound ***S*-5** to compound ***S*-1** comprises
treating compound ***S*-5** with a chlorinating agent to prepare compound ***S*-9** 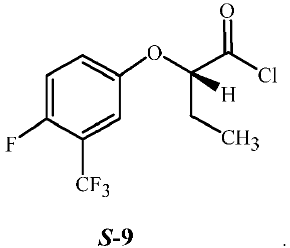 and treating compound ***S*-9** optionally in the presence of an additional base with compound **10**

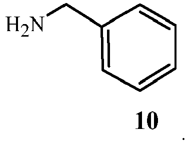

Embodiment C15. The method of Embodiment C14 wherein the chlorinating agent is POCl₃, SOCl₂, (COCl)₂ or COCl₂.

Embodiment C16. The method of Embodiment C15 wherein the chlorinating agent is thionyl chloride, i.e. SOCl₂.

Embodiment C17. The method of any of Embodiments C14 through C16 wherein the additional base comprises a hydride, alkoxide, hydroxide, carbonate, bicarbonate of an alkali metal or an alkaline earth metal; or an amide base; or a tertiary amine.

Embodiment C18. The method of Embodiment C17 wherein the additional base comprises triethylamine, sodium hydroxide, potassium hydroxide, sodium bicarbonate, potassium bicarbonate, sodium carbonate or potassium carbonate.

Embodiment C19. The method of Embodiment C18 wherein the additional base comprises triethylamine.

Embodiment D1. The method of Embodiment D wherein the chiral complex comprises ruthenium complexed with a chiral bisphosphine.

Embodiment D2. The method of Embodiment D1 wherein the chiral complex comprises a dichloro ruthenium complex with a chiral bisphosphine.

Embodiment D3. The method of Embodiment D2 wherein the chiral complex comprises a dichloro ruthenium complex with an asymmetric atropisomeric bisphosphine.

Embodiment D4. The method of any of Embodiments D through D2 wherein the bisphosphine ligand is selected from the group consisting of (*S*)-synphos, (*S*)-P-phos, (*S*)-Cl-MeO-BIPHEP, (-)-TMBTP and (*S*)-BINAP.

Embodiment D5. The method of Embodiment D4 wherein the chiral complex is chloro[(*S)*-(-)-2,2'-bis(diphenylphosphino)-1,1'-binaphthyl](*p*-cymene)ruthenium(II) chloride, i.e. (*S*)-BINAP.

Embodiment D6. The method of any of Embodiments D through D5 wherein the tertiary amine comprises triethylamine, diisopropylethylamine, *N*,*N*,*N'*,*N'*-tetramethylethylenediamine, *N*-methylpiperidine, *N*-phenylpiperidine or *N*-methylmorpholine.

Embodiment D7. The method of Embodiment D6 wherein the tertiary amine comprises triethylamine.

Embodiment D8. The method of any of Embodiments D through D7 comprising treating compound **2** with triethylamine to provide the triethylamine salt of Formula **3** 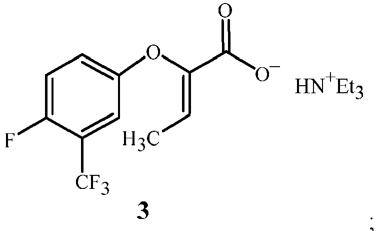 treating the triethylamine salt of Formula **3** with a hydrogen source in the presence of a chiral complex to provide the triethylamine salt ***S*-4** 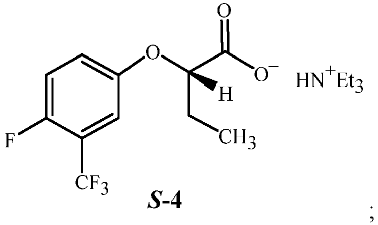 and treating the triethylamine salt ***S*-4** with acid to provide compound ***S*-5.**

Embodiment D9. The method of any of Embodiments D through D8 wherein compound **2** is prepared by treating a compound of Formula **6** 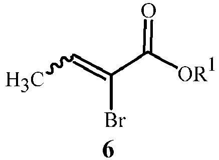 wherein R¹ is C₁-C₆ alkyl;
with compound **7** 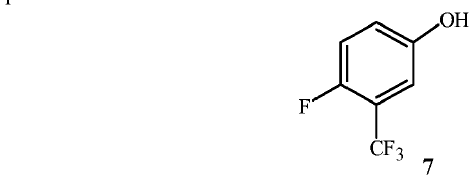 in the presence of a base to provide a compound of Formula 8 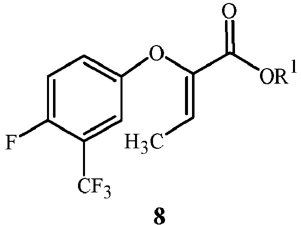 wherein R¹ is C₁-C₆ alkyl; and
hydrolyzing the compound of Formula **8.**

Embodiment D10. The method of Embodiment D9 wherein R¹ is CH₃.

Embodiment E1. The compound of Embodiment E wherein G is OH [i.e. *(Z)*-2-(4-fluoro-3-(trifluoromethyl)phenoxy)but-2-enoic acid)].

Embodiment E2. The compound of Embodiment E wherein G is OCH₃ [i.e. methyl *(Z)*-2-(4-fluoro-3-(trifluoromethyl)phenoxy)but-2-enoate)].

Embodiment E3. The compound of Embodiment E wherein G is O⁻ (ethyl)₃NH⁺.

Embodiments of this invention, including Embodiments A1 through A20, B1 through B20, C1 through C19, D1 through D10, and E1 through E3 above as well as any other embodiments described herein (including Embodiments P1 through P11), can be combined in any manner, and the descriptions of variables in the embodiments pertain not only to compounds ***S*-1, *S*-5** and a compound of Formula **16,** and the methods for their preparation, but also to the starting compounds and intermediate compounds ***S*-5** and **2,** and compounds of Formulae **6, 8, 11** and **12,** useful for preparing compound ***S*-1.**

Preferred Embodiments include the following.

Embodiment P1. The method of any of Embodiments A, B, C or D wherein the chiral complex comprises ruthenium complexed with a chiral bisphosphine.

Embodiment P2. The method of any of Embodiments A, B, C or D wherein the chiral complex is chloro[(*S*)-(-)-2,2'-bis(diphenylphosphino)-1,1'-binaphthyl](*p-*cymene)ruthenium(II) chloride, i.e. (*S*)-BINAP.

Embodiment P3. The method of any of Embodiments A, B, C or D wherein the tertiary amine comprises triethylamine.

Embodiment P4. The method of any of Embodiments A, B, C or D wherein compound ***S*-5** is prepared by the method comprising treating compound **2** 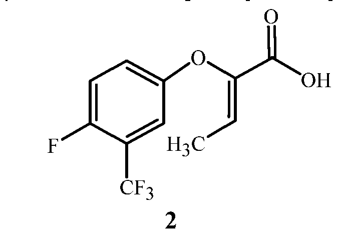 with triethylamine to provide the triethylamine salt of Formula **3** 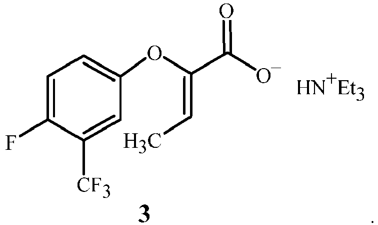 treating the triethylamine salt of Formula **3** with a hydrogen source in the presence of a chiral complex to provide the triethylamine salt of Formula ***S*-4** 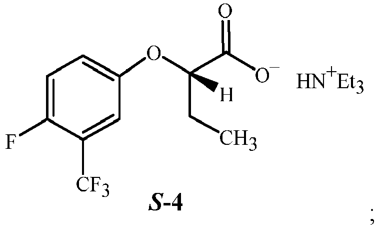 and treating the triethylamine salt of Formula ***S*-4** with acid.

Embodiment P5. The method of any of Embodiments A, B, C or D wherein the compound of Formula **2** is prepared by treating a compound of Formula **6** 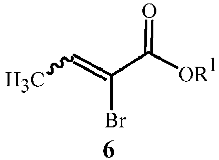 wherein R¹ is C₁-C₆ alkyl;
with compound **7** 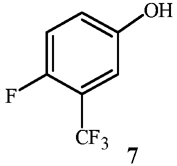 in the presence of a base to provide a compound of Formula **8** 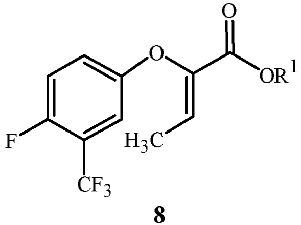 wherein R¹ is C₁-C₆ alkyl; and
hydrolyzing the compound of Formula **8.**

Embodiment P6. The method of Embodiment P5 wherein R¹ is CH₃.

Embodiment P7. The method of any of Embodiments A, B or C wherein converting compound ***S*-5** to compound ***S*-1** comprises
treating compound ***S*-5** with a chlorinating agent to prepare compound ***S*-9** 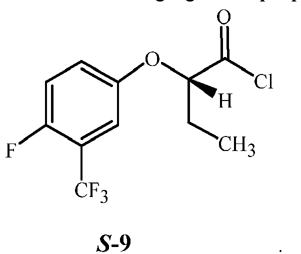 and treating compound ***S*-9** with compound **10** optionally in the presence of an additional base

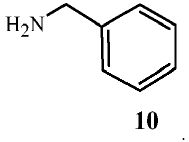

Embodiment P8. The method of Embodiment P7 wherein the chlorinating agent is thionyl chloride, i.e. SOCl₂.

Embodiment P9. The method of Embodiments P7 or P8 wherein the additional base comprises triethylamine.

Embodiment P10. The compound of Embodiment E wherein G is OH or OCH₃.

Embodiment P11. The compound of Embodiment E wherein G is O⁻ (ethyl)₃NH⁺.

Preferred Embodiments include the following.

In the following Schemes the definition of R¹ in compounds of Formulae **6, 8, 11** and **12** below are as defined above in the Summary of the Invention and description of embodiments unless otherwise indicated. The methods described herein provide and efficient and robust synthesis of compound ***S*-1.**

Obtaining organic acids of high enantiomeric purity can be accomplished in several ways, including catalytic asymmetric synthesis, chromatographic resolution, extraction resolution, membrane resolution, enzymatic resolution and diastereomeric salt resolution. The various resolution techniques depend on separation of a racemic mixture of the acid, limiting their efficiency to a maximum of 50% yield unless the undesired enantiomer can be racemized or epimerized in conjunction with the resolution. In contrast, because the precursor prochiral alkene is non-chiral at the desired asymmetric center, it is possible that the entire precursor can be converted to the desired enantiomer using catalytic asymmetric synthesis. However, catalytic asymmetric synthesis depends on good coordination between the substrate and the chiral complex to achieve high enantiomeric excess, which may be difficult to achieve.

As summarized in Scheme 1, compound ***S*-1** can be prepared from compound ***S*-5,** i.e. *(S)*-2-(4-fluoro-3-(trifluoromethyl)phenoxy)butanoic acid, wherein the compound of Formula ***S*-5** is obtained by asymmetric hydrogenation of compound **2,** i.e. *(Z)*-2-(4-fluoro-3-(trifluoromethyl)phenoxy)but-2-enoic acid, as described in greater detail with reference to Schemes 2 and 3. Catalytic asymmetric hydrogenation of compound **2** can be achieved by treatment with a hydrogen source in the presence of a chiral complex, such as wherein the chiral complex comprises ruthenium complexed with a chiral bisphosphine, including a dichloro ruthenium complex with a chiral bisphosphine. The "hydrogen source" can be any moiety that provides the equivalent of hydrogen (dihydrogen or H₂). The "hydrogen source" includes hydrogen gas or a hydrogen transfer agent. Transfer hydrogenation is the addition of hydrogen to a molecule from a source other than gaseous H₂. Hydrogen transfer agents include ammonium formate, which decomposes to ammonia, CO₂ and H₂ under suitable conditions. Another hydrogen transfer agent is isopropanol, which provides hydrogen by conversion to acetone under suitable conditions. Conversion of compound ***S*-5** to compound ***S*-1** can be effected by any of several reaction sequences subsequently described herein.

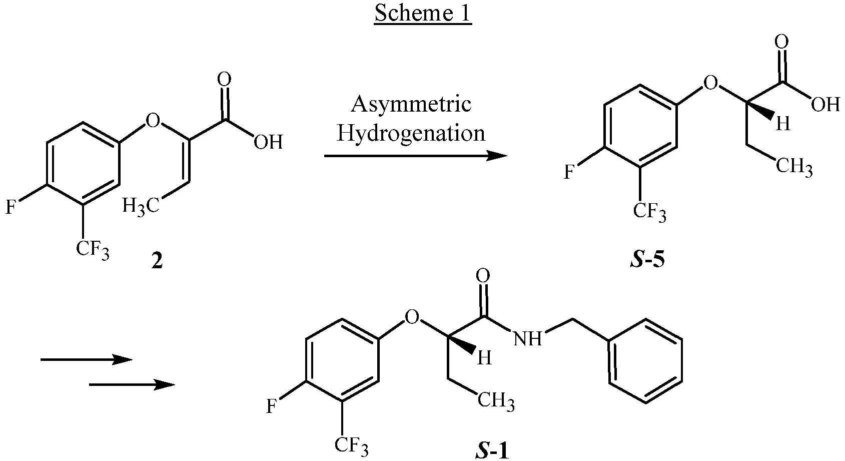

Catalytic asymmetric hydrogenation of compound **2** to provide compound ***S*-5** can be achieved with high efficiency using ruthenium complexes with atropisomeric bisphosphines. Preferred bisphosphine ligands include (*S*)-synphos, (*S*)-P-phos, (-)-TMBTP, (*S*)-Cl-MeO-BIPHEP and (*S*)-BINAP, more preferably (*S*)-BINAP, i.e. (*S*)-(2,2'-bis(diphenylphosphino)-1,1'-binaphthyl (shown in Scheme 2). For the reaction to proceed at an acceptable rate, about one equivalent of a tertiary amine such as triethylamine, diisopropylethylamine, *N*,*N*,*N'*,*N'-*tetramethylethylenediamine, *N-*methylpiperidine, *N*-phenylpiperidine or *N-*methylmorpholine, preferably triethylamine, is added. Alternatively, polymer-bound amines may be used. Suitable solvents include alcohols, optionally mixed with water, such as methanol, ethanol and isopropanol, polar aprotic solvents such as acetonitrile and ethyl acetate, and mixtures thereof. Preferably methanol is used. For similar asymmetric hydrogenations, see Org. Letters, 2004, 6, 3147-3150 and Org. Proc. Res. & Dev. 2009, 13, 525-534.

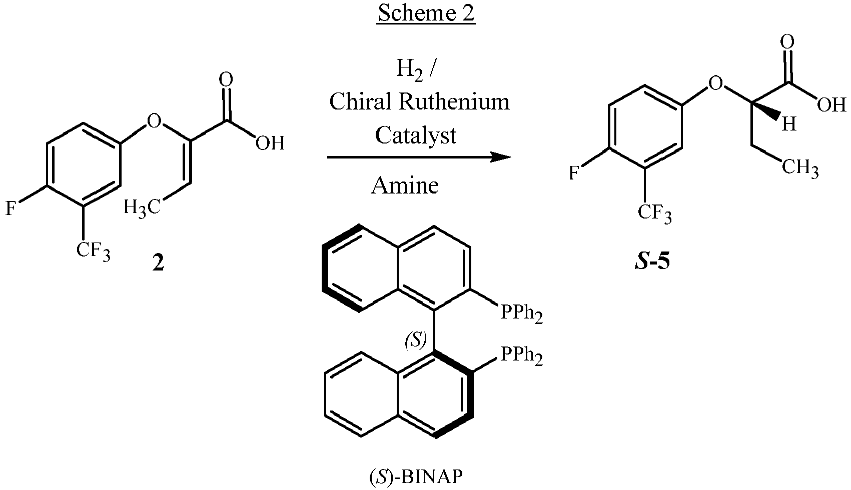

A preferred embodiment is shown in Scheme 3. Treatment of compound **2** in methanol provides the triethylamine salt **3.** Asymmetric hydrogenation using a Ru complex with (*S*)-BINAP provides the triethylamine salt ***S*-4.** Sequential treatment with base, then acid provides compound ***S*-5.**

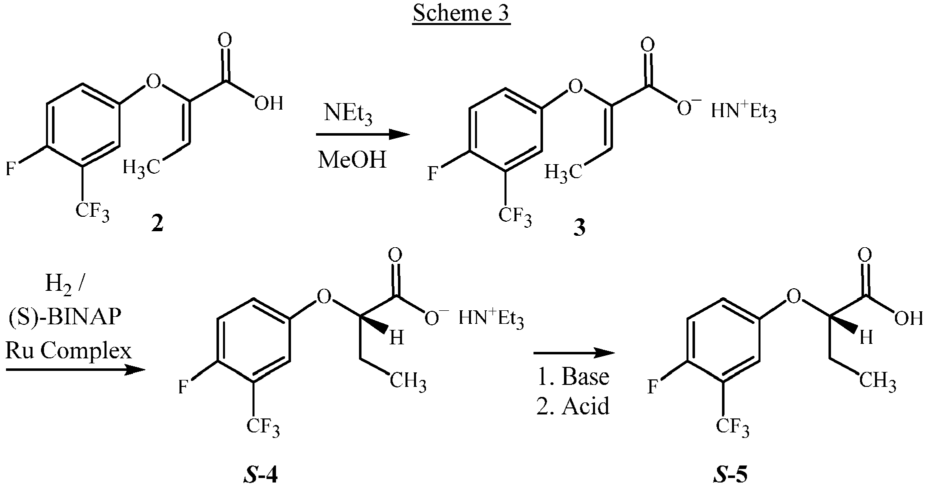

One can appreciate that the procedures summarized in Schemes 2 and 3 can be used to obtain compound ***R*-5,** if desired, with equal efficiency if the *R*-enantiomers of the atropic bisphosphines are used.

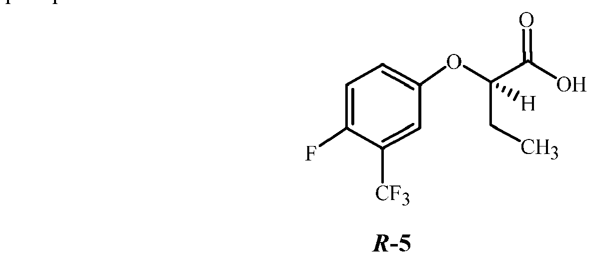

As shown in Scheme 5, a compound of Formula **6** can be treated with compound **7** (i.e. 4-fluoro-3-(trifluoromethyl)phenol) in the presence of a base to provide a compound of Formula **8.** The compound of Formula **6** may be a mixture of *E*- and *Z*-isomers. The compound of Formula **8** is typically isolated as the *Z*-isomer even if the compound of Formula **6** is a mixture of isomers. In compounds of Formulae **6** and **8,** R¹ is C₁-C₆ alkyl, preferably C₁-C₄ alkyl, more preferably C₁-C₂ alkyl, even more preferably CH₃ (i.e. the compound of Formula **8** is methyl *(Z)*-2-(4-fluoro-3-(trifluoromethyl)phenoxy)but-2-enoate). Suitable solvents include acetonitrile, dichloroethane, toluene, isopropanol, tetrahydrofuran, dimethyl sulfoxide or *N*,*N*-dimethylformamide. Preferred solvents include dichloroethane, toluene, acetonitrile or *N*,*N*-dimethylformamide, more preferably acetonitrile. Suitable bases for the reaction include a hydride, alkoxide, hydroxide, carbonate, bicarbonate of an alkali metal or an alkaline earth metal, or an amide base, or a tertiary amine. Such bases include alkali metal hydrides such as sodium hydride; or alkali metal alkoxides such as sodium isopropoxide and potassium *tert*-butoxide; or alkali metal hydroxides such as potassium hydroxide and sodium hydroxide; or alkali metal carbonates and bicarbonates such as sodium bicarbonate, potassium bicarbonate, sodium carbonate, potassium carbonate and cesium carbonate; or amide bases such as lithium bis(trimethylsilyl)amide, sodium bis(trimethylsilyl)amide and lithium diisopropylamide; or tertiary amines such as triethylamine and diisopropylethylamine. Preferred bases include sodium hydroxide, potassium hydroxide, sodium bicarbonate, potassium bicarbonate, sodium carbonate or potassium carbonate, optionally wherein the base is in an aqueous solution. More preferred is potassium carbonate.

The compound of Formula **8** can be hydrolyzed to compound **2** by treatment with aqueous base followed by acidification. Preferred bases include sodium hydroxide, potassium hydroxide, sodium bicarbonate, potassium bicarbonate, sodium carbonate or potassium carbonate, as an aqueous solution. More preferred is potassium hydroxide solution. Preferred acids for the acidification include hydrochloric, hydrobromic or sulfuric acids; more preferred is hydrochloric acid.

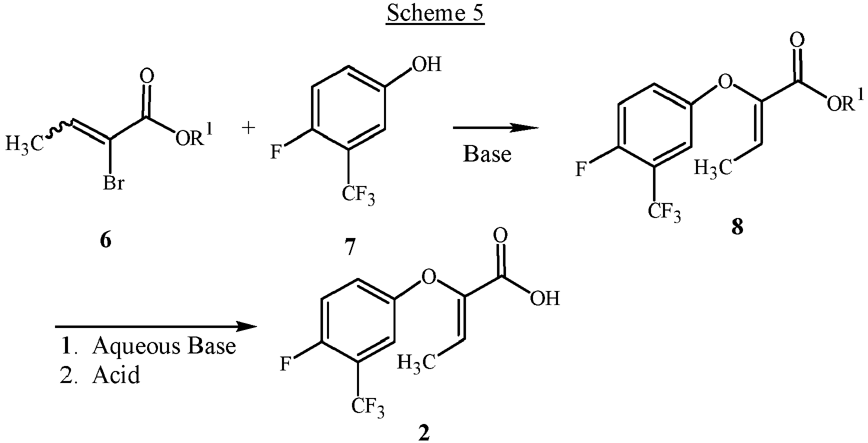

As shown in Scheme 6, the compound of Formula **6** can be prepared from a crotonate ester of Formula **11** by bromination to provide the compound of Formula **12,** followed by dehydrobromination. In compounds of Formulae **11** and **12,** R¹ is C₁-C₆ alkyl, preferably C₁-C₄ alkyl, more preferably C₁-C₂ alkyl, even more preferably CH₃. Preferably, the bromination is run in the absence of solvent. The crude product of Formula **12** can be dehydrobrominated by treatment with base, optionally in a suitable solvent. Preferred bases include sodium hydroxide, potassium hydroxide, sodium bicarbonate, potassium bicarbonate, sodium carbonate or potassium carbonate. More preferred is potassium carbonate. Suitable solvents include acetonitrile, tetrahydrofuran, dimethyl sulfoxide or *N*,*N*-dimethylformamide, preferably acetonitrile. A mixture of *E*- and *Z*-isomers of the compound of Formula **6** can be formed in the dehydrobromination.

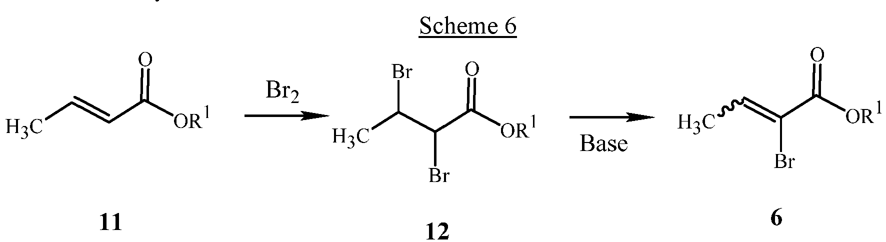

In some embodiments, dehydrobromination of the compound of Formula **12** and reaction of the compound of Formula **6** with compound **7** can be conducted sequentially in the same base/solvent system without isolation of the compound of Formula **6** to provide the compound of Formula **8.** A notable base/solvent system for such an embodiment is a slurry of solid potassium carbonate in acetonitrile.

Alternatively, compound **2** can be prepared from compound **7** as shown in Scheme 7. Allylation of compound **7** with a compound of Formula **13** wherein X is a leaving group such as Cl, Br, I or mesylate (methanesulfonate) provides compound **14.** Suitable solvents include acetonitrile, dichloromethane, dichloroethane, toluene, tetrahydrofuran, dimethyl sulfoxide or *N*,*N*-dimethylformamide. Preferred solvents include dichloromethane, dichloroethane, toluene, acetonitrile or *N*,*N*-dimethylformamide. Suitable bases for the reaction include alkali metal hydrides such as sodium hydride; or alkali metal alkoxides such as sodium isopropoxide and potassium *tert*-butoxide; or alkali metal hydroxides such as potassium hydroxide and sodium hydroxide; or alkali metal carbonates and bicarbonates such as sodium bicarbonate, potassium bicarbonate, sodium carbonate, potassium carbonate and cesium carbonate; or amide bases such as lithium bis(trimethylsilyl)amide, sodium bis(trimethylsilyl)amide and lithium diisopropylamide; or tertiary amines such as triethylamine and diisopropylethylamine. Preferred bases include sodium hydroxide, potassium hydroxide, sodium bicarbonate, potassium bicarbonate, sodium carbonate or potassium carbonate, optionally wherein the base is in an aqueous solution. More preferred is potassium carbonate.

Compound **14** can be converted to compound **2** by deprotonative alumination with an aluminum ate compound such as *i*Bu₃Al(TMP)Li followed by NHC-copper-catalyzed carboxylation of the resulting aryloxy allylaluminum species. Regio- and stereo-selective isomerization of the compound of Formula **15** can be achieved by treatment with a catalytic amount of a hindered base such as DBU. See Org. & Biomol. Chem. 2017, 15, 2370-2375 for transformations of this type.

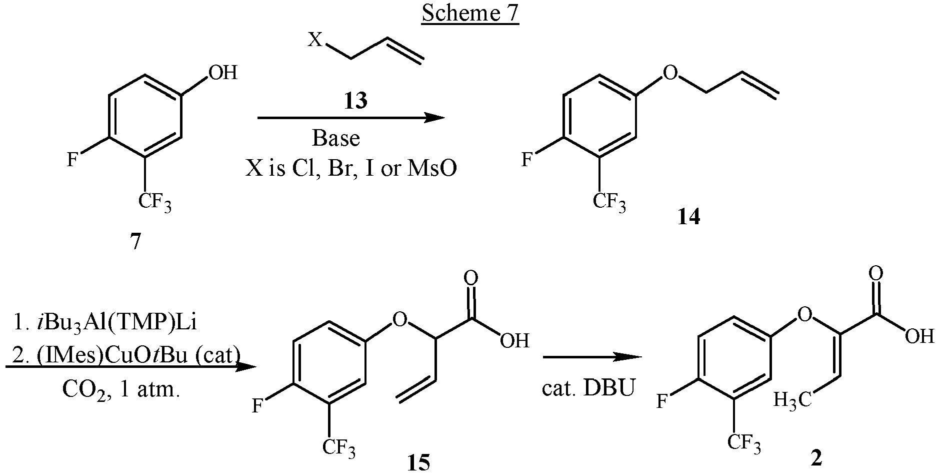

As shown in Scheme 8, compound ***S*-5,** prepared as in Scheme 5, can be converted to compound ***S*-1** by treatment with a chlorinating agent to prepare compound ***S*-9** followed by treatment with compound **10,** i.e. benzyl amine. Suitable chlorinating agents include POCl₃, SOCl₂, (COCl)₂ or COCl₂. Thionyl chloride, SOCl₂, is a preferred chlorinating agent. Suitable solvents include acetonitrile, dichloroethane, toluene, tetrahydrofuran, dimethyl sulfoxide or *N*,*N*-dimethylformamide. Preferred solvents include *N*,*N*-dimethylformamide, dichloroethane, toluene or acetonitrile, more preferably toluene.

Compound ***S*-9** can be treated with compound **10,** optionally in the presence of an additional base, to provide compound ***S*-1.** Suitable solvents include acetonitrile, dichloromethane, dichloroethane, toluene, tetrahydrofuran, dimethyl sulfoxide or *N*,*N*-dimethylformamide. Preferred solvents include dichloromethane, dichloroethane, toluene or acetonitrile, more preferably dichloromthane or toluene, most preferably dichloromethane. Suitable additional bases for the reaction include alkali metal hydrides such as sodium hydride; or alkali metal alkoxides such as sodium isopropoxide and potassium *tert-*butoxide; or alkali metal hydroxides such as potassium hydroxide and sodium hydroxide; or alkali metal carbonates and bicarbonates such as sodium bicarbonate, potassium bicarbonate, sodium carbonate, potassium carbonate and cesium carbonate; or bases such as lithium bis(trimethylsilyl)amide, sodium bis(trimethylsilyl)amide and lithium diisopropylamide; or tertiary amines such as triethylamine and diisopropylethylamine. Preferred additional bases include triethylamine, sodium hydroxide, potassium hydroxide, sodium bicarbonate, potassium bicarbonate, sodium carbonate or potassium carbonate, more preferably triethylamine.

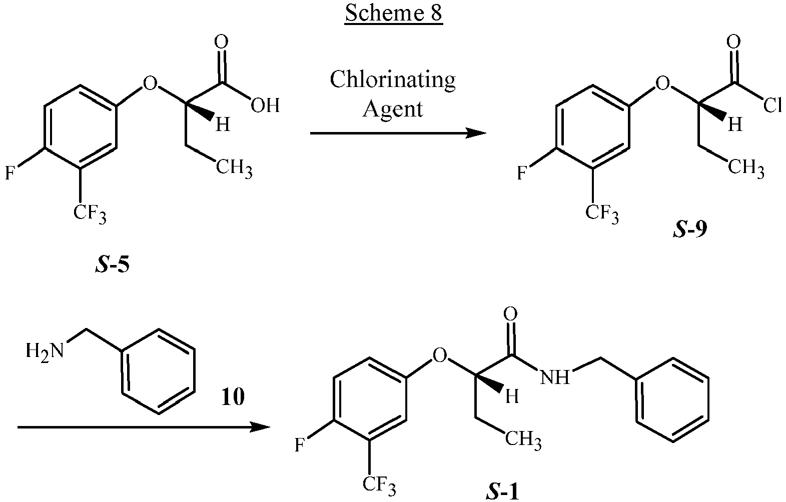

Alternatively, as shown in Scheme 9, compound ***S*-5** can be treated with compound **10** to prepare compound ***S*-1.** Preferably, the treatment comprises heating compound ***S*-5** with about 2 to 5 molar equivalents of compound **10,** such as about three equivalents, at about 100 to 125 °C, such as about 110 to 120 °C. Optionally, a solvent such as toluene can be used. The crude material obtained after removal of excess benzyl amine can be recrystallized from a mixture of isopropanol and water to provide compound ***S*-1.**

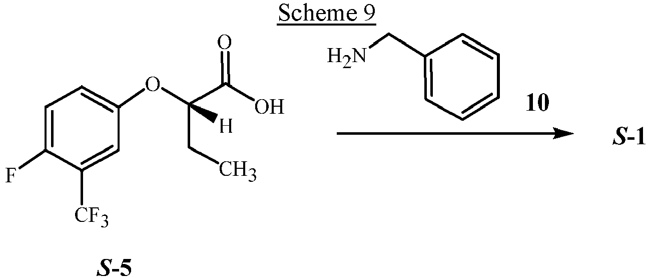

In some embodiments, each of compounds of Formulae **6, 8, 11** and **12** or compounds **2, 3, *S*-4, *S*-5, *S*-9, 14** and **15** can be isolated after preparation and before being carried into the next step. Alternatively, two or more of the steps from compounds **7, 10, 11** to compound ***S*-1** can be combined without isolating the respective intermediate compounds. For example, compound **2** can be converted to compound ***S*-5** without isolating the intermediate amine salts **3** and ***S*-4.**

It is recognized that some reagents and reaction conditions described above for preparing the compounds may not be compatible with certain functionalities present in the intermediates. In these instances, the incorporation of protection/deprotection sequences or functional group interconversions into the synthesis will aid in obtaining the desired products. The use and choice of the protecting groups will be apparent to one skilled in chemical synthesis (see, for example, Greene, T. W.; Wuts, P. G. M. Protective Groups in Organic Synthesis, 2nd ed.; Wiley: New York, 1991). One skilled in the art will recognize that, in some cases, after the introduction of a given reagent as it is depicted in any individual scheme, it may be necessary to perform additional routine synthetic steps not described in detail to complete the synthesis of the compounds. One skilled in the art will also recognize that it may be necessary to perform a combination of the steps illustrated in the above schemes in an order other than that implied by the particular sequence presented to prepare the compounds. One skilled in the art will also recognize that the compounds and the intermediates described herein can be subjected to various electrophilic, nucleophilic, radical, organometallic, oxidation, and reduction reactions to add substituents or modify existing substituents.

Without further elaboration, it is believed that one skilled in the art using the preceding description can utilize the present invention to its fullest extent. The following Examples are, therefore, to be construed as merely illustrative and not limiting of the disclosure in any way whatsoever. Steps in the following Examples illustrate a procedure for each step in an overall synthetic transformation, and the starting material for each step may not have necessarily been prepared by a particular preparative run whose procedure is described in other Examples or Steps. Percentages are by weight. The abbreviation "h" stands for "hour" or "hours". The abbreviation "GC" stands for "gas chromatography" or "gas chromatographic". Gas chromatographic analysis used generally known procedures selected to provide sufficient resolution of a compound of interest from other compounds to allow for determination of its purity and identification, optionally using known standards for comparison. The abbreviation "mp" stands for melting point.

### SYNTHESIS EXAMPLE 1

### Preparation of (S)-2-(4-fluoro-3-(trifluoromethyl)phenoxy)butanoic acid

### Step 1 Preparation of methyl 2-bromocrotonate.

To a jacketed reactor fitted with a mechanical stirrer, condenser and thermometer pocket was charged methyl crotonate (58.8 g, 582 mmol), and the temperature was adjusted to about 25 °C. Bromine (98.7 g, 612 mol) was added over a period of about 2 h, so that the temperature was maintained at about 25 to 35 °C. During the addition, the reaction mixture changed from a clear colorless solution to a clear red solution that darkened over time. Bromine fumes were observed. The progress of the reaction was monitored by taking 100 uL aliquots which were separated between ethyl acetate (2 mL) and aqueous HCl (2 mL). The organic phase was analyzed by GC. The reaction was continued until methyl crotonate was less than 0.3% by GC area. The crude methyl 2,3-dibromobutanoate was removed from the reactor. To the jacketed reactor was charged acetonitrile (157 g) and potassium carbonate (179 g, 1280 mmol) and the temperature was brought to about 70 °C. The crude methyl 2,3-dibromobutanoate was added to the reactor over a 4-h period with vigorous stirring. The slurry changed from dark red to yellow-orange. The reaction mixture was stirred at about 70 °C until GC analysis showed methyl-2,3-dibromobutanoate to be less than 0.3% by GC area. The reaction was not worked up but used as the resulting slurry in Step 2. If worked up by filtration, washing of the filter cake with acetonitrile and evaporation of volatiles, the crude methyl-2-bromocrotonate can be obtained as an oil. Expected yield is greater than 97% with a purity of about 95%.

### Step 2: Preparation of methyl (Z)-2-(4-fluoro-3-(trifluoromethyl)phenoxy)but-2-enoate.

To the jacketed reactor containing the slurry from Step 1 was charged 4-fluoro-3-(trifluoromethyl)phenol (91.5 g, 503 mmol) over about 2 h with vigorous stirring at 70 °C. Stirring was continued until 4-fluoro-3-(trifluoromethyl)phenol was less than 0.2% by GC area. About 8 h in total was needed for completion. The slurry was cooled to about 25 °C and then filtered. The filter cake was washed with additional acetonitrile (1 x 60 g, optionally additional washes). The combined filtrates were heated in the reactor to about 70 to 75 °C and the pressure brought to about 100 mbar to distill off the acetonitrile to obtain the title compound as an oil. The relatively high jacket temperature avoids solidification of the product, which has a mp of 55-56 °C. Methyl tert-butyl ether (MTBE) (164 g) was added and the jacket temperature was brought to about 25 °C. The dissolution of the product is endothermic, thus cooling the solution. Purity by GC analysis was about 97-98 %. The solution of the title compound in MTBE was carried into the next step without further purification or characterization. If desired, the product can be isolated by removing the MTBE to provide a solid that can be recrystallized from acetonitrile.

### Step 3: Preparation of (Z)-2-(4-fluoro-3-(trifluoromethyl)phenoxy)but-2-enoic acid.

To a jacketed reactor fitted with a mechanical stirrer, condenser and thermometer pocket was charged 50% potassium hydroxide (67.5 g, 601 mmol), and the temperature was adjusted to about 50 °C. The solution of the title compound of Step 2 in MTBE was added to the reactor over a 1-h period. After the addition was complete, the reaction mass was stirred until the starting ester was less than 0.5% by GC area, about 30 min. The pH was checked to be at least 9 before proceeding. Water (67.5 g) was added to the reactor, followed by addition of 37% HCl (71 g) over a 1-h period. The phases were separated and the organic phase was weighed to establish the concentration, assuming the only components were the title product and MTBE. Hexane (164 g) was added to the reactor, to provide a 30% w/w mixture of MTBE and hexane, and the mixture was seeded with crystals of the title compound (2 g) from a previous preparation. The mixture was cooled linearly from 50 to -10 °C during a 12-h period. The resulting slurry was filtered and the filter cake was air dried to provide the title product (103 g) in 80 to 85% yield. The filter cake was not washed, to avoid re-dissolving some of the title product. Purity by GC analysis was about 99%. The crude solid was carried into the next step without further purification or characterization.

### Step 4: Preparation of triethylammonium (S)-2-(4-fluoro-3-(trifluoromethyl)phenoxy)butanoate.

To a jacketed reactor, fitted with a mechanical stirrer, thermometer and a dropping funnel, was charged methanol (about 150 g) and the title product of Step 3 (103 g, 385 mmol). The temperature of the reaction mass was adjusted to about 20 °C and triethylamine (56.4 g, 557 mmol) was added over a 30-min period while maintaining the temperature at about 20 °C. After the addition was complete, the mixture was filtered to remove tiny black particles. The filtered solution was charged to a hydrogenation reactor fitted with a hollow-shaft stirrer to efficiently disperse hydrogen gas into the liquid phase. A solution of *(S)*-RuCl[p-cymene(BINAP)]Cl (644 mg, 0.69 mmol) in methanol (about 14 g) was charged to the reactor. The reactor was sealed and stirring started. The reactor was purged thrice by pressurizing to 4 bar of nitrogen, holding for two minutes and venting the nitrogen. The reactor was purged thrice by pressurizing to 4 bar of hydrogen, holding for two minutes and venting the hydrogen into a flow of nitrogen. The reaction mass was adjusted to 55 °C and the hydrogen pressure was increased to 9 bar with vigorous stirring. The mixture was maintained under hydrogen pressure until uptake of hydrogen ceased (about 2 h), and the hydrogen was vented into a flow of nitrogen. Conversion can be monitored in-line using an appropriate hydrogen flowmeter. Alternatively, sampling and ¹H-NMR analysis can be used. The temperature was adjusted to ambient temperature and the resulting solution was used charged to a jacketed reactor, fitted with a mechanical stirrer, condenser with receiver, thermometer and a dropping funnel, with the solution obtained from Step 4. The temperature was adjusted to about 55 °C and the pressure was reduced to about 10 mbar to distill off volatiles to obtain the crude product of the title compound as a brown oil, having an enantiomeric ratio of about 95:5 ratio of *(S):(R)*, ee of about 90%.

### Step 5: Preparation of (S)-2-(4-fluoro-3-(trifluoromethyl)phenoxy)butanoic acid.

The reactor containing the crude product of the title compound of Step 4 was brought to about 25 °C and charged with dichloromethane (177 g). Hydrochloric acid (61.4 g, 522 mmol) was added over 10 minutes, maintaining the temperature at about 25 °C. The pH was checked to ensure it was about 0 to 1, and water (41.4 g) was added. The phases were separated to remove the aqueous phase. The pressure in the reactor was reduced to about110 mbar and the dichloromethane was distilled into the receiver. The jacket temperature was brought to about 50 °C while maintaining gentle condensation of dichloromethane into the receiver. The pressure was adjusted to 10 mbar to ensure complete removal of the dichloromethane. After removal of dichloromethane, the reactor was brought to ambient temperature and pressure to provide the title compound, which was not further purified or characterized before being carried into the next step.

### SYNTHESIS EXAMPLE 2

### Preparation of (S)-N-benzyl-2-(4-fluoro-3-trifluoromethylphenoxy)-butanoic amide

### Step 1: Preparation of (S)-2-(4-fluoro-3-(trifluoromethyl)phenoxy)butanoic acid chloride.

To the reactor containing *(S)*-2-(4-fluoro-3-(trifluoromethyl)phenoxy)butanoic acid (about 385 mmol), i.e. the title material from Synthesis Example 1, Step 5, was added dichloromethane (177 g). The receiver was removed and the condenser was configured to perform as reflux condenser. A caustic scrubber was attached and pyridine (0.9 g, 11 mmol) was added. Thionyl chloride (52.1 g, 438 mmol) was added over about 10 minutes, with evolution of white fumes. The reactor jacket was adjusted to about 50-55 °C so that the reaction mass was brought to reflux at about 40 °C. Sulfur dioxide and hydrogen chloride off-gassing was routed through the caustic scrubber. The reflux was continued for about 3 to 5 h, until the amount of the starting acid was less than about 0.5% by GC analysis of an aliquot. The pressure in the reactor was reduced to about1 10 mbar and the volatiles were distilled off. The pressure was adjusted to 10 mbar to ensure complete removal of all volatiles. The reactor was brought to ambient temperature and pressure under nitrogen and the reactor was fitted with a calcium chloride-packed drying tube to provide the title compound as an oil. The crude material was used directly in the next step without further purification or characterization.

### Step 2: Preparation of (S)-N-benzyl-2-(4-fluoro-3-trifluoromethylphenoxy)-butanoic amide.

To the reactor containing *(S)*-2-(4-fluoro-3-(trifluoromethyl)phenoxy)butanoic acid chloride (about 385 mmol), i.e. the title material from Step 1, was added dichloromethane (177 g). The solution was charged to a jacketed reactor, fitted with a mechanical stirrer, thermometer and a dropping funnel and the reaction mixture was cooled to about 5 °C. A mixture of benzylamine (43.2 g, 438 mmol) and triethylamine (44.3, 438 mmol) was added via the dropping funnel over a period of about 1 h while maintaining the temperature below 10 °C. The reaction during addition was highly exothermic. After the addition was complete, the reaction mixture was stirred for about 30 minutes, until the amount of the starting acid chloride was less than about 0.5% by GC analysis of an aliquot. The temperature was adjusted to about 5 °C and water (131.8) was added. After 10 minutes, the phases were separated, and the aqueous layer was removed. The volatiles, mostly dichloromethane, were removed by lowering the pressure to ca. 100 mbar and then incrementally increasing the reaction mass temperature to 70 °C. Isopropanol (200 g) was added and the resulting mixture was cooled to about 40 °C and stirred for about 15 minutes. Water (90.1 g) was added to provide a mixture that corresponds to a solution of the title compound (about 30% w/w) in an isopropanol: water mixture of 7:3. The solution was seeded with (S)-N-benzyl-2-(4-fluoro-3-trifluoromethylphenoxy)-butanoic amide (2 g, 2% seeding amount) from a previous run and then cooled using the following cooling profile

| Time (h) | Temperature (°C) |
|---|---|
| 0 | 40 |
| 20 | 25 |
| 24 | 5 |

The resulting slurry was filtered at 5 °C and the cold filtrate was used to wash the reactor and the filter cake. The filter cake was not washed any further to avoid re-dissolving the product. The filter cake was dried on the filter under suction and further air-dried to provide the title compound as an off-white solid with purity of about 95% and isolated yield of about 75 to 80% from (S)-2-(4-fluoro-3-(trifluoromethyl)phenoxy)butanoic acid.

## Claims

1. A method for preparing compound ***S*-1** 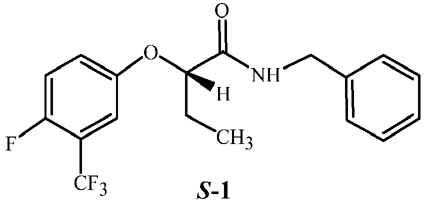 from compound ***S*-5** 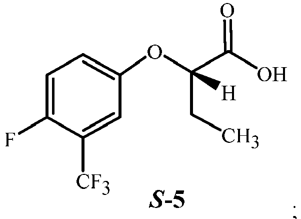 wherein compound ***S*-5** is prepared by treating compound **2** 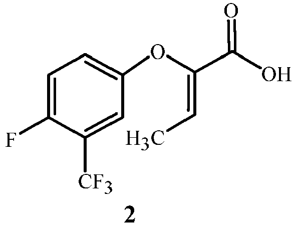 with a tertiary amine and a hydrogen source in the presence of a chiral complex.

2. The method of Claim 1 wherein compound ***S*-5** is prepared by the method comprising treating compound **2** 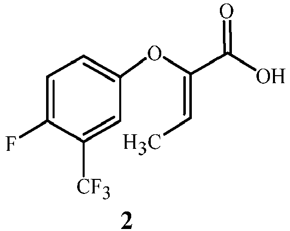 with triethylamine to provide the triethylamine salt of Formula **3** 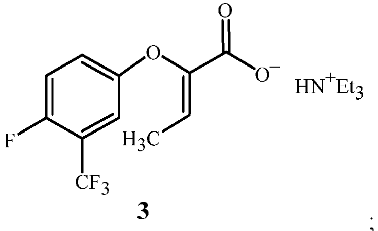 treating the triethylamine salt of Formula **3** with the hydrogen source in the presence of the chiral complex to provide the triethylamine salt of Formula ***S*-4** 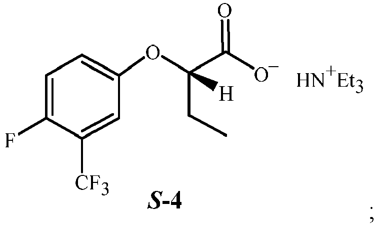 and treating the triethylamine salt of Formula ***S*-4** with acid.

3. The method of Claim 1 wherein compound ***S*-5** is converted to the compound of Formula ***S*-1** by the method comprising
treating compound ***S*-5** with a chlorinating agent to prepare compound ***S*-9** 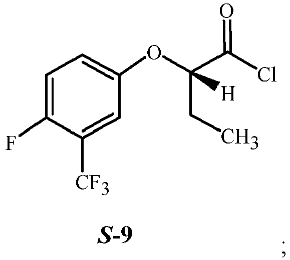 and
treating compound ***S*-9** optionally in the presence of an additional base with compound **10**

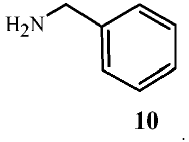

4. The method of Claim 3 wherein the chlorinating agent is thionyl chloride.

5. The method of Claim 3 wherein the additional base comprises triethylamine.

6. A method for preparing compound ***S*-5** 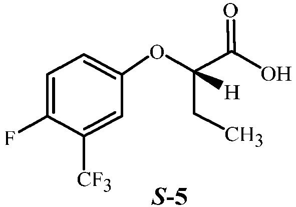 the method comprising:
treating compound **2** 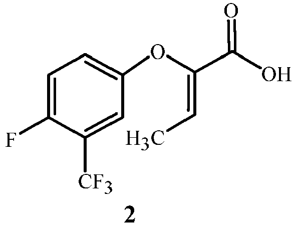 with a tertiary amine and a hydrogen source in the presence of a chiral complex.

7. The method of Claim 1 or Claim 6 wherein the chiral complex comprises ruthenium complexed with a chiral bisphosphine.

8. The method of Claim 7 wherein the chiral complex is chloro[(*S*)-(-)-2,2'-bis(diphenylphosphino)-1,1'-binaphthyl](*p*-cymene)ruthenium(II) chloride.

9. The method of Claim 1 or Claim 6 wherein the tertiary amine comprises triethylamine.

10. The method of Claim 6 wherein compound ***S*-5** is prepared by the method comprising treating compound **2** 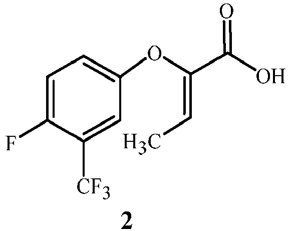 with triethylamine to provide the triethylamine salt of Formula **3** 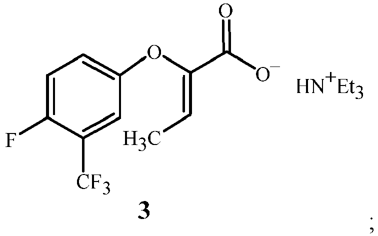 treating the triethylamine salt of Formula **3** with the hydrogen source in the presence of the chiral complex to provide the triethylamine salt of Formula ***S*-4** 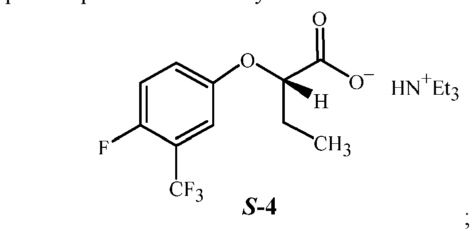 and
treating the triethylamine salt of Formula ***S*-4** with acid.

11. The method of Claim 1 or Claim 6 wherein compound **2** is prepared by treating a compound of Formula **6** 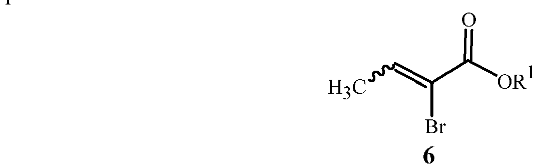 wherein R¹ is C₁-C₆ alkyl;
with compound **7** 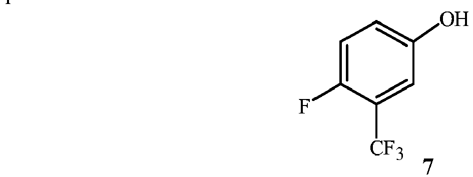 in the presence of a base to provide a compound of Formula **8** 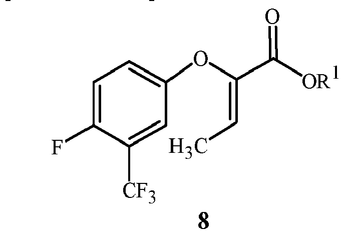 wherein R¹ is C₁-C₆ alkyl; and
hydrolyzing the compound of Formula **8.**

12. The method of Claim 11 wherein R¹ is CH₃.

13. A compound of Formula **16** 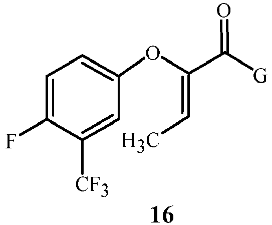 wherein G is OH, C₁-C₆ alkoxy or O⁻ M⁺;
and M⁺ is an alkali metal cation or a tertiary ammonium cation.

14. The compound of Claim 13 wherein G is OH or OCH₃.

15. The compound of Claim 13 wherein G is O⁻ (ethyl)₃NH⁺.

## Patentansprüche

1. Verfahren zur Herstellung der Verbindung ***S*-1** 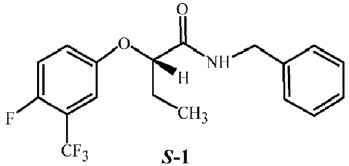 aus der Verbindung ***S*-5** 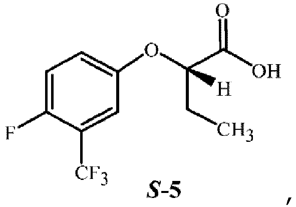 wobei die Verbindung ***S*-5** hergestellt wird, indem man die Verbindung **2** 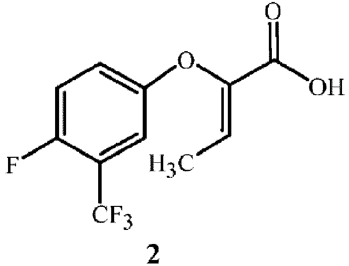 in Gegenwart eines chiralen Komplexes mit einem tertiären Amin und einer Wasserstoffquelle umsetzt.

2. Verfahren nach Anspruch 1, wobei die Verbindung ***S*-5** hergestellt wird nach dem Verfahren, welches die Behandlung der Verbindung **2** 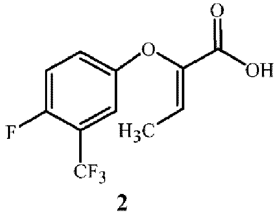 mit Triethylamin unter Bereitstellung des Triethylaminsalzes der Formel **3** 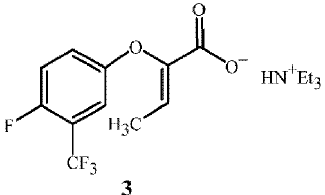 die Behandlung des Triethylaminsalzes der Formel **3** mit der Wasserstoffquelle in Gegenwart des chiralen Komplexes unter Bereitstellung des Triethylaminsalzes der Formel ***S*-4** 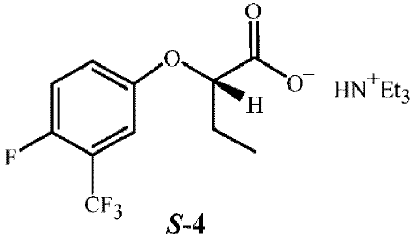 und
die Behandlung des Triethylaminsalzes der Formel ***S*-4** mit Säure umfasst.

3. Verfahren nach Anspruch 1, wobei Verbindung ***S*-5** durch das Verfahren, das die Behandlung der Verbindung ***S*-5** mit einem Chlorierungsmittel zur Herstellung der Verbindung ***S*-9** 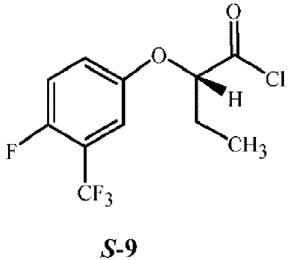 und die Behandlung der Verbindung ***S*-9,** gegebenenfalls in Gegenwart einer zusätzlichen Base, mit der Verbindung **10** 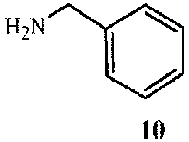 umfasst, in die Verbindung der Formula ***S*-1** überführt wird.

4. Verfahren nach Anspruch 3, wobei es sich bei dem Chlorierungsmittel um Thionylchlorid handelt.

5. Verfahren nach Anspruch 3, wobei die zusätzliche Base Triethylamin umfasst.

6. Verfahren zur Herstellung der Verbindung ***S*-5** 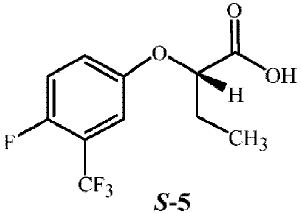 wobei das Verfahren Folgendes umfasst:
die Behandlung der Verbindung **2** 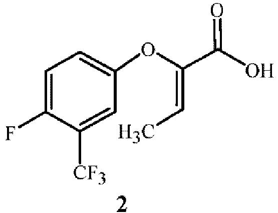 mit einem tertiären Amin und einer Wasserstoffquelle in Gegenwart eines chiralen Komplexes.

7. Verfahren nach Anspruch 1 oder Anspruch 6, wobei der chirale Komplex Ruthenium komplexiert mit einem chiralen Bisphosphin umfasst.

8. Verfahren nach Anspruch 7, wobei es sich bei dem chiralen Komplex um Chlor[(*S*)-(-)-2,2'-bis(diphenyl-phosphino)-1,1'-binaphthyl](*p*-cymol)ruthenium(II)-chlorid handelt.

9. Verfahren nach Anspruch 1 oder Anspruch 6, wobei das tertiäre Amin Triethylamin umfasst.

10. Verfahren nach Anspruch 6, wobei Verbindung ***S*-5** durch das Verfahren hergestellt wird, das die Behandlung der Verbindung **2** 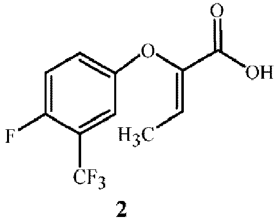 mit Triethylamin unter Bereitstellung des Triethylaminsalzes der Formel **3** 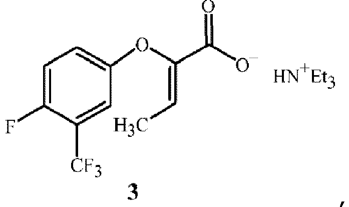 die Behandlung des Triethylaminsalzes der Formel **3** mit der Wasserstoffquelle in Gegenwart des chiralen Komplexes unter Bereitstellung des Triethylaminsalzes der Formel ***S*-4** 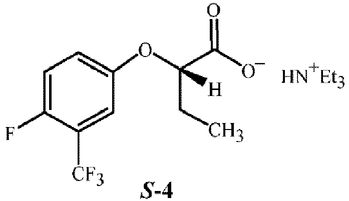 und
die Behandlung des Triethylaminsalzes der Formel ***S*-4** mit Säure umfasst.

11. Verfahren nach Anspruch 1 oder Anspruch 6, wobei die Verbindung **2** hergestellt wird, indem man eine Verbindung der Formel **6** 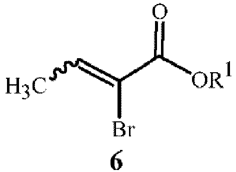 wobei R¹ für C₁-C₆-Alkyl steht, in Gegenwart einer Base mit Verbindung **7** 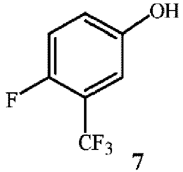 behandelt, unter Bereitstellung einer Verbindung der Formel **8** 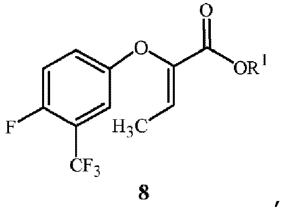 wobei R¹ für C₁-C₆-Alkyl steht, und
die Verbindung der Formel **8** hydrolysiert.

12. Verfahren nach Anspruch 11, wobei R¹ für CH₃ steht.

13. Verbindung der Formel **16** 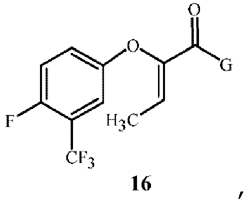 wobei G für OH, C₁-C₆-Alkoxy oder O⁻M⁺ steht und M⁺ für ein Alkalimetallkation oder ein tertiäres Ammoniumkation steht.

14. Verbindung nach Anspruch 13, wobei G für OH oder OCH₃ steht.

15. Verbindung nach Anspruch 13, wobei G für O⁻ (Ethyl)₃NH⁺ steht.

## Revendications

1. Procédé pour la préparation du composé *S*-1 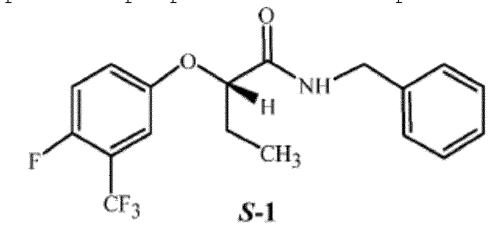 à partir du composé *S*-5 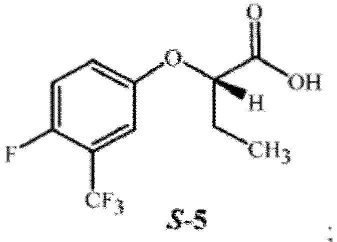 le composé 5-5 étant préparé par traitement du composé 2 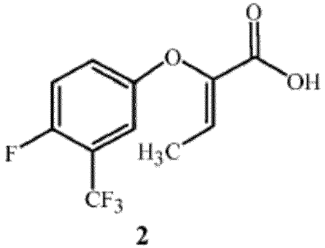 avec une amine tertiaire et une source d'hydrogène en la présence d'un complexe chiral.

2. Procédé selon la revendication 1, le composé *S*-5 étant préparé par le procédé comprenant le traitement du composé 2 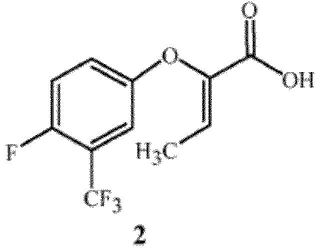 avec de la triéthylamine pour fournir le sel de triéthylamine de formule 3 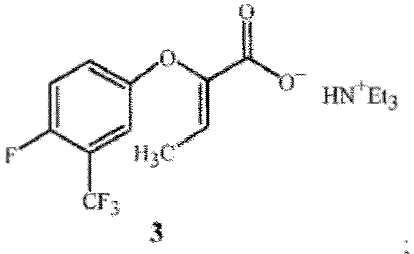 le traitement du sel de triéthylamine de formule 3 avec la source d'hydrogène en la présence du complexe chiral pour fournir le sel de triéthylamine de formule *S*-4 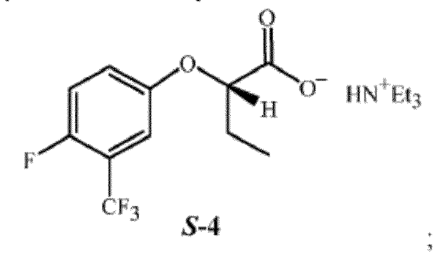 et
le traitement du sel de triéthylamine de formule *S-*4 avec un acide.

3. Procédé selon la revendication 1, le composé *S*-5 étant converti en composé de formule *S*-1 par le procédé comprenant
le traitement du composé 5-5 avec un agent de chloration pour préparer le composé *S*-9 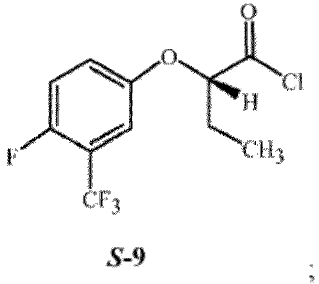 et
le traitement du composé *S*-9 éventuellement en la présence d'une base supplémentaire avec le composé 10

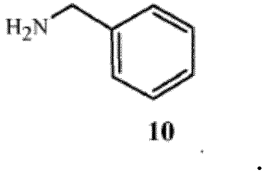

4. Procédé selon la revendication 3, l'agent de chloration étant le chlorure de thionyle.

5. Procédé selon la revendication 3, la base supplémentaire comprenant la triéthylamine.

6. Procédé pour la préparation du composé *S*-5 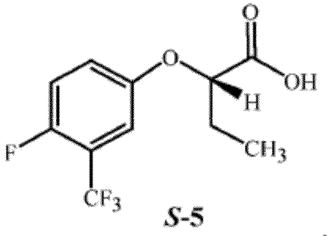 le procédé comprenant :
le traitement du composé 2 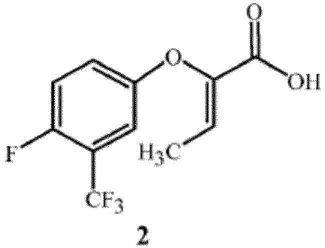 avec une amine tertiaire et une source d'hydrogène en la présence d'un complexe chiral.

7. Procédé selon la revendication 1 ou la revendication 6, le complexe chiral comprenant du ruthénium complexé par une bisphosphine chirale.

8. Procédé selon la revendication 7, le complexe chiral étant le chlorure de chloro[(*S*)-(-)-2,2'-bis(diphénylphosphino)-1,1'-binaphtyl][(*p-*cymène)ruthénium(II).

9. Procédé selon la revendication 1 ou la revendication 6, l'amine tertiaire comprenant la triéthylamine.

10. Procédé selon la revendication 6, le composé S-5 étant préparé par le procédé comprenant le traitement du composé 2 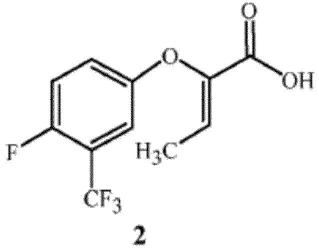 avec la triéthylamine pour fournir le sel de triéthylamine de formule 3 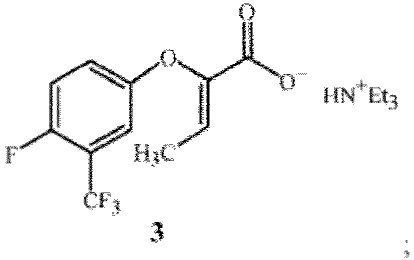 le traitement du sel de triéthylamine de formule 3 avec la source d'hydrogène en la présence du complexe chiral pour fournir le sel de triéthylamine de formule *S*-4 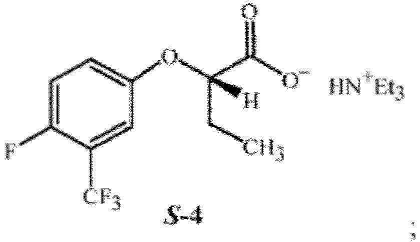 et
le traitement du sel de triéthylamine de formule *S-*4 avec un acide.

11. Procédé selon la revendication 1 ou la revendication 6, le composé 2 étant préparé par traitement d'un composé de formule 6 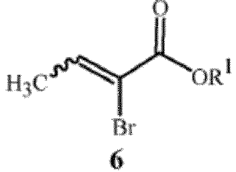 R¹ étant C₁₋₆ alkyle ;
avec le composé 7 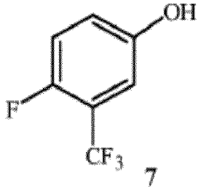 en la présence d'une base pour fournir un composé de formule 8 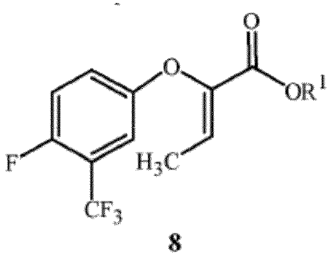 R¹ étant C₁₋₆ alkyle ; et
l'hydrolyse du composé de formule 8.

12. Procédé selon la revendication 11, R¹ étant CH₃.

13. Composé de formule 16 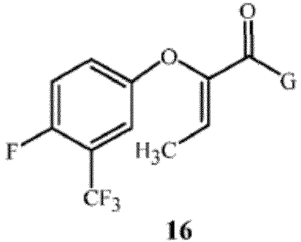 G étant OH, C₁₋₆ alcoxy ou O⁻M⁺ ;
et M⁺ étant un cation de métal alcalin ou un cation ammonium tertiaire.

14. Composé selon la revendication 13, G étant OH ou OCH₃.

15. Composé selon la revendication 13, G étant O⁻ (éthyl)₃NH⁺.
